(19) Europäisches Patentamt
European Patent Office
Office européen des brevets

(11) Veröffentlichungsnummer: **0 318 882 B1**

(12) # EUROPÄISCHE PATENTSCHRIFT

(45) Veröffentlichungstag der Patentschrift: **23.06.93**

(51) Int. Cl.[5]: **C07C 323/22**, C07C 317/24, C07D 273/04, A01N 47/34, A01N 43/88

(21) Anmeldenummer: **88119765.1**

(22) Anmeldetag: **26.11.88**

(54) **(Thio)benzoylharnstoffe und deren funktionelle Derivate, Verfahren zu ihrer Herstellung, sie enthaltende Mittel und ihre Verwendung als Schädlingsbekämpfungsmittel.**

(30) Priorität: **04.12.87 DE 3741062**

(43) Veröffentlichungstag der Anmeldung:
**07.06.89 Patentblatt 89/23**

(45) Bekanntmachung des Hinweises auf die Patenterteilung:
**23.06.93 Patentblatt 93/25**

(84) Benannte Vertragsstaaten:
**AT BE CH DE ES FR GB GR IT LI NL**

(56) Entgegenhaltungen:
**EP-A- 0 084 652**
**EP-A- 0 285 428**
**GB-A- 2 106 500**
**US-A- 4 399 152**

**CHEMICAL ABSTRACTS, Band 109, Nr. 21, 21. November 1988, Seite 262, Zusammenfassung Nr. 185494z, Columbus, Ohio, US; & JP-A-63156765**

(73) Patentinhaber: **HOECHST AKTIENGESELLSCHAFT**
**Postfach 80 03 20**
**W-6230 Frankturt am Main 80(DE)**

(72) Erfinder: **Koch, Volker, Dr.**
**Altkönigstrasse**
**W-6233 Kelkheim (Taunus)(DE)**
Erfinder: **Schnatterer,Stefan, Dr.**
**Flurscheideweg 11**
**W-6230 Frankturt am Main 80(DE)**
Erfinder: **Bonin, Werner, Dr.**
**Im Schulzehnten 18**
**W-6233 Kelkheim (Taunus)(DE)**
Erfinder: **Kern, Manfred, Dr.**
**Im Traminer Weg 8**
**W-6501 Lörzweiler(DE)**
Erfinder: **Knauf, Werner, Dr.**
**Im Kirschgarten 24**
**W-6239 Eppstein/Taunus(DE)**
Erfinder: **Waltersdorfer, Anna, Dr.**
**Rauenthaler Weg 28**
**W-6000 Frankturt am Main 71(DE)**

EP 0 318 882 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**JOURNAL OF AGRICULTURAL AND FOOD CHEMISTRY, Band 21, Nr. 3, 1973, Seiten 348-354, Washington, US; K. WELLINGA et al.: "Synthesis and laboratory evaluation of 1-(2,6-distributed benzoyl)-3-phenylureas, a new class of insecticides. I. 1-(2,6-dichlorobenzoyl)-3-phenylureas"**

## Beschreibung

Es ist bekannt, daß bestimmte (Thio)benzoylharnstoffe und deren funktionelle Derivate insektizide und akarizide Eigenschaften aufweisen, s. DE-OS 25 37 413, EP-A 84 652, EP-A 5944.

Diese besitzen jedoch teilweise unzureichende Wirksamkeiten.

Es wurden neue substituierte (Thio)benzoylharnstoffe und von diesen abgeleitete funktionelle Derivate mit vorteilhaften schädlingsbekämpfenden Eigenschaften gefunden.

Gegenstand der vorliegenden Erfindung sind daher die Verbindungen der Formel I

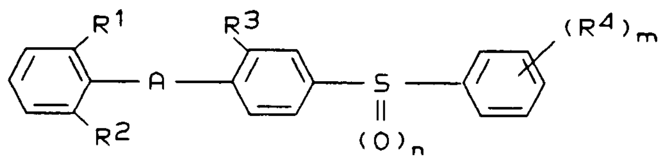

(I),

worin A einen Rest der Formeln $A^1$, $A^2$ oder $A^3$

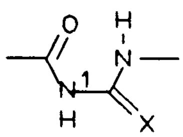

($A^1$)

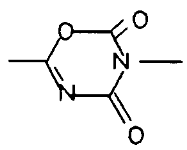

($A^2$)

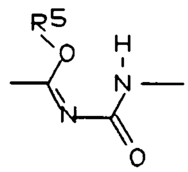

($A^3$)

,

| | |
|---|---|
| $R^1$ bis $R^3$ | jeweils unabhängig voneinander Wasserstoff oder Halogen, |
| $R^4$ | jeweils unabhängig von einander Halogen, $(C_1\text{-}C_3)$Halogenalkyl, oder Nitro, |
| $R^5$ | $(C_1\text{-}C_5)$Alkyl, das halogeniert sein kann, |
| X | Sauerstoff oder Schwefel, |
| n | eine Zahl von 0 bis 2 und |
| m | eine Zahl von 1 bis 4 |

bedeuten, mit der Maßgabe, daß $(R^4)_m$ nicht ausschließlich Halogen oder nicht ausschließlich Nitro bedeutet, sowie im Fall $A = A^1$ deren in der Landwirtschaft einsetzbaren Salze.

Halogen bedeutet F, Cl, Br oder I, insbesonders F oder Cl. Unter halogeniert bzw. Halo sind ebenfalls die vorgenannten Substituenten zu verstehen. Halogenalkyl bedeutet insbesondere $CF_3$.

Bevorzugte Vebindungen der Formel I sind solche, bei denen $R^1$ = Cl, F, $R^2$ = H, F; $R^3$ = H oder F, $R^4$ = jeweils unabhängig voneinander Cl, F, $CF_3$ oder Nitro; $R^5$ = Ethyl; X = Sauerstoff; n = 0 und m = 1 bis 3 bedeuten, sowie im Fall A = $A^1$ deren in der Landwirtschaft einsetzbaren Salze.

Besonders bevorzugte Verbindungen der Formel I sind solche, bei denen $R^1$ = Cl, F und $R^2$ = H, F; $R^3$ = H oder F, $(R^4)_m$ = 2-Cl, 4-$CF_3$; $R^5$ = Ethyl; X = Sauerstoff; n = 0 und m = 1 oder 2 bedeuten, sowie im Fall A = $A^1$ deren in der Landwirtschaft einsetzbaren Salze.

Von den beiden letztgenannten Gruppen sind solche Verbindungen von besonderer Bedeutung, bei denen A einen Rest der Formel $A^1$ oder $A^3$ bedeutet. Hierbei sind folgende Verbindungen besonders zu erwähnen:
N-(2-Chlorbenzoyl)-N'-(4-(2-chlor-4-trifluormethyl-1-phenylmercapto)phenyl)harnstoff,
N-(2-Fluorbenzoyl)-N'-(4-(2-chlor-4-trifluormethyl-1-phenylmercapto)phenyl)harnstoff,
N-(2,6-Difluorbenzoyl)-N'-(4-(2-chlor-4-trifluormethyl-1-phenylmercapto)phenyl)harnstoff,
N-(2-Chlorbenzoyl)-N'-(4-(2-chlor-4-trifluormethyl-1-phenylmercapto)-2-fluorphenyl)harnstoff,
N-(2-Fluorbenzoyl)-N'-(4-(2-chlor-4-trifluormethyl-1-phenylmercapto)-2-fluorphenyl)harnstoff,
N(2,6-Difluorbenzoyl)-N'-(4-(2-chlor-4-trifluormethyl-1-phenylmercapto)-2-fluorphenyl)harnstoff,
N-(N-(2-Chlor-4-trifluormethyl-1-phenylmercapto)phenyl)carbamoyl)-2-chlorbenzcarboximidsäureethylester,
N-(N-(2-Chlor-4-trifluormethyl-1-phenylmercapto)phenyl)carbamoyl)-2-fluorbenzcarboximidsäureethylester,
N-(N-(2-Chlor-4-trifluormethyl-1-phenylmercapto)phenyl)carbamoyl)-2,6-difluorbenzcarboximidsäureethylester.

Die Erfindung umfaßt auch alle Stereoisomeren und deren Gemische der Verbindungen der Formel I, wie die E- und Z-Isomeren bei ungesättigten Strukturen oder optische Isomere, falls Chiralitätszentren

auftreten. Ferner können die Verbindungen der Formel I je nach Substitution in tautomeren Formel vorliegen, die ebenfalls von der Erfindung umfaßt werden.

Ebenfalls umfaßt die Erfindung Salze der Verbindungen der Formel I für A = $A^1$, die durch Deprotonierung am $N^1$-Atom gebildet werden können.

Als Salze der Verbindungen der Formel I kommen die in der Landwirtschaft einsetzbaren Salze infrage, insbesondere die Alkali-, Erdalkali-, Ammonium- oder ein- bis vierfach durch organische Reste (wie Alkyl, Hydroxyalkyl) substituierte Ammoniumsalze.

Gegenstand der vorliegenden Erfindung sind auch Verfahren zur Herstellung der Verbindungen der Formel I, dadurch gekennzeichnet, daß man

a) für A = $A^1$

$a_1$) eine Verbindung der Formel II

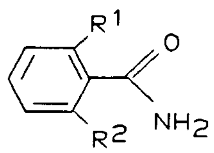

(II),

worin $R^1$ und $R^2$ die Bedeutungen wie in Formel I besitzen, mit einer Verbindung der Formel III

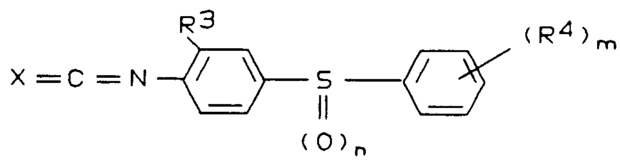

(III),

worin X, $R^3$, $R^4$, m und n die Bedeutungen wie in Formel I besitzen, umsetzt oder

$a_2$) eine Verbindung der Formel IV

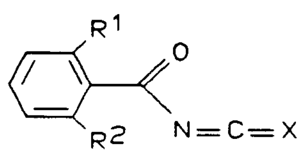

(IV),

worin $R^1$, $R^2$ und X die Bedeutungen wie in Formel I besitzen, mit einer Verbindung der Formel V

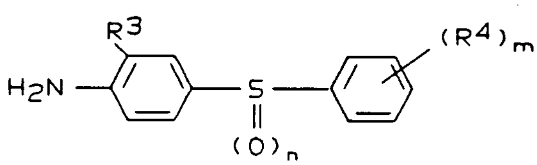

(V),

worin $R^3$, $R^4$, m und n die Bedeutungen wie in Formel I besitzen, umsetzt

oder

b) für A = $A^2$

eine Verbindung der Formel III mit einer Verbindung der Formel IV, wobei X jeweils 0 bedeutet, umsetzt

oder

c) für A = $A^3$

$c_1$) eine Verbindung der Formel VI

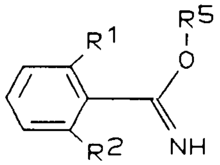

(VI),

worin $R^1$, $R^2$ und $R^5$ die Bedeutungen wie in Formel I besitzen, mit einer Verbindung der Formel III umsetzt
oder
$c_2$) eine Verbindung der Formel VII

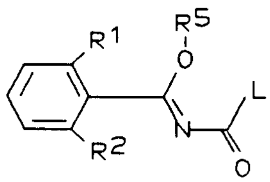

(VII),

worin $R^1$, $R^2$ und $R^5$ die Bedeutungen wie in Formel I besitzen und L für eine nucleofuge Abgangsgruppe steht, mit einer Verbindung der Formel V umsetzt.

Als Beispiele für die nucleofuge Abgangsgruppe L seien genannt:
Halogen, ($C_1$-$C_3$)Alkylthio, ($C_1$-$C_3$)Haloalkoxy, Triazolyl oder Imidazolyl.

Die Herstellung der Benzamide II und Benzoyliso(thio)cyanate IV ist literaturbekannt (vgl. J. Agr. Food Chem. 21, 348 und 993 (1973); Houben-Weyl E4, S. 806 und S. 869).
Die Iso(thio)cyanate III erhält man nach literaturbekannten Methoden aus den Anilinen der Formel V (Houben-Weyl E4, S. 738 und S. 834).

Die Aniline der Formel V lassen sich analog zu literaturbekannten Verfahren herstellen und zwar

a) durch Umsetzung entsprechender 4-Mercaptonaniline mit substituierten Halogenbenzolen (Houben-Weyl E 11/1, S. 175)

b) durch Umsetzung ggf. substituierter Nitrothiophenole mit substituierten Halobenzolen (wobei diese Thiodiphenylether dann zu Sulfoxiden und Sulfonen oxidiert werden können, s. Houben-Weyl E 11/1, S. 702 ff und E 11/2, S. 1194 ff) und anschließender Reduktion der Nitrogruppe (Houben-Weyl E 11/1, S. 175 und 4/1c, S. 506 ff und 742)

c) durch Umsetzung substituierter Thiophenole mit Halonitrobenzolen (wobei diese Thiodiphenylether dann zu Sulfoxiden und Sulfonen oxidiert werden können, (vgl. b)) und anschließender Reduktion der Nitrogruppe (vgl.b)). Die Verbindungen der Formel V sind zum Teil neu und insofern ebenfalls Gegenstand der vorliegenden Erfindung.

Die Imidate der Formel VI sind literaturbekannt (DE-OS 35 14 450).

Die Verbindungen der Formel VII lassen sich nach literaturbekannten Methoden aus den Imidaten der Formel VI herstellen (EP-A 135 894).

Die Reaktionsbedingungen (z.B. Lösungsmittel, Reaktionstemperatur, Katalysatoren) für die Herstellung und Isolierung der Verbindungen der Formel I sind literaturbekannt:

| | | |
|---|---|---|
| **Verfahrensvariante** | $a_1$), $a_2$) | z.B. DE-A 21 23 236 |
| | b) | z.B. US-PS 4 150 158 |
| | $c_1$), $c_2$) | z.B. EP-A 135 894 |

Die Wirkstoffe eignen sich bei guter Pflanzenverträglichkeit und günstiger Warmblutertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu

den oben erwähnten Schädlingen gehören:
Aus der Ordnung der Acarina z.B. Acarus siro, Agras spp., Ornithrodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.
Aus der Ordnung der Isopoda, z.B. Oniscus asselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregarai.
Aus der Ordnung der Dermaptera z.B. Forficula auricularia.
Aus der Ordnung des Isoptera z.B. Reticulitermes spp.
Aus der Ordnung der Anoplura z.B. Phylloera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporarium, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Naphotettix cinciticeps, Lecanium corni, Saissetia oleae, Laodelphax striatellus, Nilaparvata lugens, Aonidiella auroantii, Apidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cocoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrhynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon slstritialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliophora erythrocephala, Lucilia spp., Chrysomyia spp., Cuterebra spp., Gastrophilus spp., Hypobosca spp., Stomoxys spp., Oestrus spp. Hypoderma spp., Tabanus spp., Tannia spp., Bibio hortulanus, Oscinella frit, Phorbia spp., Pegomyia hyoscyami, Ceratitis capitata, Dacus oleae, Tripula paludosa.
Aus der Ordnung der Siphonaptera z.B. Xenopsylla cheopsis, Ceratophyllus spp.
Aus der Ordnung der Arachnida z.B. Scorpio maurus, Latrodectus mactans.
Aus der Klasse der Helminthen z.B. Haemonchus, Trichostrongulus, Ostertagia. Cooperia, Chabertia, Strongyloides, Oesophagostomum, Hyostrongulus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphinema.
Aus der Klasse der Gastropoda z.B. Deroceras spp., Arion spp., Lymnaea spp., Galba spp., Succinea spp., Biophalaria spp., Bulinus spp., Oncomelania spp.
Aus der Klasse der Bivalva z.B. Dreissena spp.

Gegenstand der Erfindung sind auch Mittel, die die Verbindungen der Formel I neben geeigneten Formulierungshilfsmittel enthalten.

Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemischphysikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro- Sprüh- Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Boods, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:

Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hänge der Wirkstoffgehalt zum Teile davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr

Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a.
Bevorzugte Mischungspartner sind

1. aus der Gruppe der Phosphorverbindungen
Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos, O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. aus der Gruppe der Carbamate
Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cumenylbutyryl(methyl)-carbamate, Oxamyl, Pirimicarb, Propoxur, Thiodicarb, Thiofanox, Ethyl, 4,6,9-triaza-4-benzyl-6,10-dimethyl-8-oxa-7-oxo-5,11-dithia-9-dodecenoate (OK 135), 1-methylthio(ethylideneamino)-N-methyl-N-(morpholinothio)carbamate (UC 51717);

3. aus der Gruppe der Carbonsäureester
Allethrin, Alphametrin, 5-Benzyl-3-furylmethyl-(E)-(1R)-cis-2,2-di-methyl-3-(2-oxothiolan-3-ylidenemethyl)-cyclopropanecarboxylate, Bioallethrin, Bioallethrin((S)-cyclopentylisomer), Bioresmethrin, Bisphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D- isomer), Permethrin, Pheothrin ((R)-Isomer), d-Pralethrin, Pyrethrine (natürliche Produkte), Resmethrin, Tefluthrin, Tetramethrin, Tralomethrin;

4. aus der Gruppe der Amidine
Amitraz, Chlordimeform;

5. aus der Gruppe der Zinnverbindungen
Cyhexatin, Fenbutatinoxide;

6. Sonstige
Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-di-phenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tachens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Nachfolgende Beispiele dienen zur Erläuterung der Erfindung.

**A. Formulierungsbeispiele**

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

**B. Chemische Beispiele**

a) Vorprodukte

4-(2-Chlor-4-trifluormethyl-1-phenylmercapto)anilin

19,86 g (0,1 Mol) 3-Chlor-4-fluorbenzotrifluorid und 20 g wasserfreies Kaliumcarbonat wurden in 40 ml absolutem Acetonitril vorgelegt und bei Raumtemperatur 12,52 g (0,1 Mol) 4-Mercaptoanilin zugetropft. Es wurde 6 h bei 40 °C gerührt, danach das Kaliumcarbonat abfiltriert und das Lösemittel im Vakuum abdestilliert. Der Rückstand wurde dann in 50 ml Diethylether aufgenommen, neutral gewaschen und nach dem Abdestillieren des Lösemittels der Rückstand aus Cyclohexan umkristallisiert.

Ausbeute: 25,2 g (83 %)
Fp.: 59-60 °C

analog wurde hergestellt
4-(2-Chlor-4-trifluormethyl-1-phenylmercapto)-2-fluoranilin
Fp.: 48 °C

4-(2-Chlor-4-trifluormethyl-1-phenylmercapto)phenylisocyanat

15,19 g (50 mMol) 4-(2-Chlor-4-trifluormethyl-1-phenylmercapto)anilin und 25 g (0,25 Mol) Phosgen in 100 ml absolutem Toluol wurden langsam von 0 °C auf Rückfluß erhitzt, das überschüssige Phosgen mit Inertgas vertrieben und das Lösemittel eingedampft. Das so erhaltene Öl wurde ohne weitere Reinigung für die Folgereaktionen eingesetzt.

Ausbeute: 15,7 g (95 %)

b) Endprodukte

Beispiel 1

N-(2-Chlorbenzoyl-N'-(4-(2-chlor-4-trifluormethyl-1-phenylmercapto)phenyl)harnstoff

1,56 g (10 mMol) 2-Chlorbenzamid und 3,30 g (10 mMol) 4-(2-Chlor-4-trifluormethyl-1-phenylmercapto)-phenylisocyanat wurden in 10 ml absolutem Toluol 3 h am Rückfluß gekocht. Nach dem Abkühlen wurden die ausgefallenen Kristalle abgesaugt, mit Toluol gewaschen und getrocknet.

Ausbeute: 4,46 g (92 %)
Fp.: 186 °C

Beispiel 2

N-(4-(2-Chlor-4-trifluormethyl-1-phenylmercapto)phenyl)-N'-(2,6-difluorbenzoyl)harnstoff

3,03 g (10 mMol) 4-(2-Chlor-4-trifluormethyl-1-phenylmercapto)anilin wurden in 5 ml absolutem n-Heptan vorgelegt, 1,83 g (10 mMol) 2,6-Difluorbenzoylisocyanat zugetropft und 2 h bei Raumtemperatur nachgerührt. Die Kristalle wurden abgesaugt und aus Toluol umkristallisiert.

Ausbeute: 4,33 g (89 %)
Fp.: 197 °C

Die in der nachfolgenden Tabelle aufgeführten Verbindungen der Formel I lassen sich in analoger Weise herstellen.

**Tabelle 1**

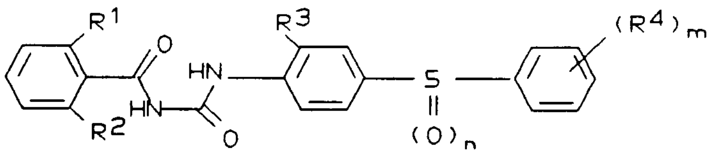

(I),

| Bsp.Nr. | $R^1$ | $R^2$ | $R^3$ | $(R^4)_m$ | n | Fp (°C) |
|---|---|---|---|---|---|---|
| 3 | F | H | H | 2-Cl, 4-$CF_3$ | 0 | 178 |
| 4 | Cl | H | H | 2-Cl, 4-$CF_3$ | 1 | 220 |
| 5 | F | H | H | 2-Cl, 4-$CF_3$ | 1 | |
| 6 | F | F | H | 2-Cl, 4-$CF_3$ | 1 | |
| 7 | Cl | H | H | 2-Cl, 4-$CF_3$ | 2 | |
| 8 | F | H | H | 2-Cl, 4-$CF_3$ | 2 | |
| 9 | F | F | H | 2-Cl, 4-$CF_3$ | 2 | |
| 10 | Cl | H | F | 2-Cl, 4-$CF_3$ | 0 | 162 |
| 11 | F | H | F | 2-Cl, 4-$CF_3$ | 0 | |
| 12 | F | F | F | 2-Cl, 4-$CF_3$ | 0 | 177 |
| 13 | Cl | H | Cl | 2-Cl, 4-$CF_3$ | 0 | 104 |
| 14 | F | H | Cl | 2-Cl, 4-$CF_3$ | 0 | |
| 15 | F | F | Cl | 2-Cl, 4-$CF_3$ | 0 | 106 |
| 16 | Cl | H | H | 2-F, 4-$CF_3$ | 0 | 151-152 |
| 17 | F | H | H | 2-F, 4-$CF_3$ | 0 | 171-172 |
| 18 | F | F | H | 2-F, 4-$CF_3$ | 0 | 190-191 |
| 19 | Cl | H | F | 2-F, 4-$CF_3$ | 0 | |
| 20 | F | H | F | 2-F, 4-$CF_3$ | 0 | |
| 21 | F | F | F | 2-F, 4-$CF_3$ | 0 | |

**Fortsetzung der Tabelle 1**

| Bsp.Nr. | $R^1$ | $R^2$ | $R^3$ | $(R^4)_m$ | n | Fp (°C) |
|---|---|---|---|---|---|---|
| 22 | Cl | H | H | 2-$CF_3$, 4-Cl | 0 | |
| 23 | F | H | H | 2-$CF_3$, 4-Cl | 0 | |
| 24 | F | F | H | 2-$CF_3$, 4-Cl | 0 | 158 |
| 25 | Cl | H | H | 3-Cl, 4-$CF_3$ | 0 | 148 |
| 26 | F | H | H | 3-Cl, 4-$CF_3$ | 0 | |
| 27 | F | F | H | 3-Cl, 4-$CF_3$ | 0 | 175 |
| 28 | Cl | H | H | 2-$CF_3$, 5-Cl | 0 | |
| 29 | F | H | H | 2-$CF_3$, 5-Cl | 0 | |
| 30 | F | F | H | 2-$CF_3$, 5-Cl | 0 | |
| 31 | Cl | H | H | 2,6-$Cl_2$, 4-$CF_3$ | 0 | 179-180 |
| 32 | F | H | H | 2,6-$Cl_2$, 4-$CF_3$ | 0 | 186-187 |
| 33 | F | F | H | 2,6-$Cl_2$, 4-$CF_3$ | 0 | 185-186 |
| 34 | Cl | H | F | 2,6-$Cl_2$, 4-$CF_3$ | 0 | |
| 35 | F | H | F | 2,6-$Cl_2$, 4-$CF_3$ | 0 | |
| 36 | F | F | F | 2,6-$Cl_2$, 4-$CF_3$ | 0 | |
| 37 | Cl | H | H | 2-$NO_2$, 4-$CF_3$ | 0 | 235-238 |
| 38 | F | H | H | 2-$NO_2$, 4-$CF_3$ | 0 | 200-202 |
| 39 | F | F | H | 2-$NO_2$, 4-$CF_3$ | 0 | 226 |
| 40 | Cl | H | H | 3-$CF_3$, 4-$NO_2$ | 0 | 168-170 |
| 41 | F | H | H | 3-$CF_3$, 4-$NO_2$ | 0 | 189 |
| 42 | F | F | H | 3-$CF_3$, 4-$NO_2$ | 0 | 205-206 |
| 43 | Cl | H | H | 2-Cl, 4-$NO_2$ | 0 | 226-227 |
| 44 | F | H | H | 2-Cl, 4-$NO_2$ | 0 | 208-209 |
| 45 | F | F | H | 2-Cl, 4-$NO_2$ | 0 | 217-218 |
| 46 | F | H | H | 2,6-$(NO_2)_2$, 4-$CF_3$ | 0 | |
| 47 | F | F | H | 2,6-$(NO_2)_2$, 4-$CF_3$ | 0 | 236-238 |
| 48 | Cl | H | H | 2,4-$(NO_2)_2$, 6-$CF_3$ | 0 | 216-219 |
| 49 | F | H | H | 2,4-$(NO_2)_2$, 6-$CF_3$ | 0 | |

**Fortsetzung der Tabelle 1**

| Bsp.Nr. | $R^1$ | $R^2$ | $R^3$ | $(R^4)_m$ | n | Fp (°C) |
|---|---|---|---|---|---|---|
| 49a | Cl | H | H | 2,6-$(NO_2)_2$, 4-$CF_3$ | 0 | 232-233 |
| 50 | F | F | H | 2,4-$(NO_2)_2$, 6-$CF_3$ | 0 | 216-217 |
| 51 | Cl | H | H | 2,6-$(NO_2)_2$, 3-Cl, 4-$CF_3$ | 0 | |
| 52 | F | H | H | 2,6-$(NO_2)_2$, 3-Cl, 4-$CF_3$ | 0 | |
| 52a | F | F | H | 2,6-$(NO_2)_2$, 3-Cl, 4-$CF_3$ | 0 | |
| 52b | Cl | H | H | 2-$CF_3$, 4-$NO_2$ | 0 | 224-226 |
| 52c | F | H | H | 2-$CF_3$, 4-$NO_2$ | 0 | 236 |
| 52d | F | F | H | 2-$CF_3$, 4-$NO_2$ | 0 | 249 |
| 52e | Cl | H | H | 3-Cl, 4-$CF_3$, 5-$NO_2$ | 0 | 242-245 |
| 52f | F | F | H | 3-Cl, 4-$CF_3$, 5-$NO_2$ | 0 | 248-250 |
| 52g | Cl | H | F | 2-Cl, 4-$CF_3$ | 2 | 165 |
| 52h | F | F | H | 4-$CF_3$ | 0 | 194 |
| 52i | Cl | H | H | 4-$CF_3$ | 0 | 173 |
| 52j | Cl | H | H | 2-Br, 4-$CF_3$ | 0 | 157 |
| 52k | Br | H | F | 2-Cl, 4-$CF_3$ | 0 | 160 |
| 52l | F | Cl | F | 2-Cl, 4-$CF_3$ | 0 | 158 |
| 52m | Cl | H | F | 2-$CF_3$, 4-$NO_2$ | 0 | 214 |
| 52n | Cl | H | F | 2-$NO_2$, 4-$CF_3$ | 0 | 238 |
| 52o | Cl | H | F | 2-Cl, 4-$NO_2$ | 0 | 197 |
| 52p | Cl | H | Br | 2-Cl, 4-$CF_3$ | 0 | 97 |
| 52q | Cl | H | F | 3-$CF_3$ | 0 | 147 |

Beispiel 53

2-(2-Chlorphenyl)-5-(4-(2-chlor-4-trifluormethyl-1-phenylmercapto)phenyl)-6H-1,3,5-oxadiazin-4,6-dion

1,81 g (10 mMol) 2-Chlorbenzoylisocyanat und 3,30 g (10 mMol) 4-(2-Chlor-4-trifluormethylphenylmercapto)phenylisocyanat wurden 16 h unter Feuchtigkeitsausschluß bei 70 °C gerührt. Nach dem Abkühlen wurde mit 5 ml absolutem n-Heptan verrührt, abgesaugt und der Feststoff aus Toluol umkristallisiert.

Ausbeute: 4.34 g (85 %)

Fp.: 193-194 °C

Analog zu der in Beispiel 53 beschriebenen Verfahrensweise lassen sich die folgenden Verbindungen der Formel I herstellen (Tabelle 2):

**Tabelle 2**

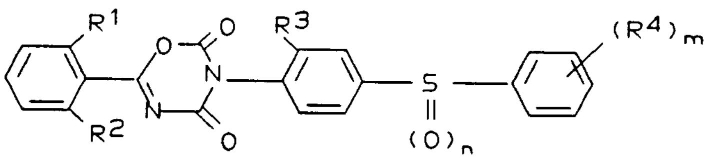

(I),

| Bsp.Nr. | $R^1$ | $R^2$ | $R^3$ | $(R^4)_m$ | n | Fp (°C) |
|---|---|---|---|---|---|---|
| 54 | F | H | H | 2-Cl, 4-$CF_3$ | 0 | 183-184 |
| 55 | F | F | H | 2-Cl, 4-$CF_3$ | 0 | 174-175 |
| 56 | Cl | H | H | 2-Cl, 4-$CF_3$ | 1 | |
| 57 | F | H | H | 2-Cl, 4-$CF_3$ | 1 | |
| 58 | F | F | H | 2-Cl, 4-$CF_3$ | 1 | |
| 59 | Cl | H | H | 2-Cl, 4-$CF_3$ | 2 | |
| 60 | F | H | H | 2-Cl, 4-$CF_3$ | 2 | |
| 61 | F | F | H | 2-Cl, 4-$CF_3$ | 2 | |
| 62 | Cl | H | F | 2-Cl, 4-$CF_3$ | 0 | |
| 63 | F | H | F | 2-Cl, 4-$CF_3$ | 0 | |
| 64 | F | F | F | 2-Cl, 4-$CF_3$ | 0 | |
| 65 | Cl | H | Cl | 2-Cl, 4-$CF_3$ | 0 | |
| 66 | F | H | Cl | 2-Cl, 4-$CF_3$ | 0 | |
| 67 | F | F | Cl | 2-Cl, 4-$CF_3$ | 0 | |
| 68 | Cl | H | H | 2-F, 4-$CF_3$ | 0 | |

**Fortsetzung der Tabelle 2**

| Bsp.Nr. | $R^1$ | $R^2$ | $R^3$ | $(R^4)_m$ | n | Fp (°C) |
|---|---|---|---|---|---|---|
| 69 | F | H | H | 2-F, 4-$CF_3$ | 0 | |
| 70 | F | F | H | 2-F, 4-$CF_3$ | 0 | |
| 71 | Cl | H | F | 2-F, 4-$CF_3$ | 0 | |
| 72 | F | H | F | 2-F, 4-$CF_3$ | 0 | |
| 73 | F | F | F | 2-F, 4-$CF_3$ | 0 | |
| 74 | Cl | H | H | 2-$CF_3$, 4-Cl | 0 | |
| 75 | F | H | H | 2-$CF_3$, 4-Cl | 0 | |
| 76 | F | F | H | 2-$CF_3$, 4-Cl | 0 | |
| 77 | Cl | H | H | 3-Cl, 4-$CF_3$ | 0 | |
| 78 | Cl | H | H | 2,6-$Cl_2$, 4-$CF_3$ | 0 | |
| 79 | F | H | H | 2,6-$Cl_2$, 4-$CF_3$ | 0 | |
| 80 | F | F | H | 2,6-$Cl_2$, 4-$CF_3$ | 0 | |
| 81 | Cl | H | F | 2,6-$Cl_2$, 4-$CF_3$ | 0 | |
| 82 | F | H | F | 2,6-$Cl_2$, 4-$CF_3$ | 0 | |

Beispiel 83

N-(N-(4-(2-Chlor-4-trifluormethyl-1-phenylmercapto)phenyl)carbamoyl)-2-chlorbenzcarboximidsäureethylester

1,83 g (10 mMol) 2-Chlorbenzcarboximidsäureethylester wurden auf 0 °C gekühlt und unter Rühren 3,30 g (10 mMol) 4-(2-Chlor-4-trifluormethylphenylmercapto)phenylisocyanat zugetropft. Nach 3 h wurden 5 ml absolutes n-Heptan zugegeben, verrührt und abgesaugt.

Ausbeute: 4,67 g (91 %)
Fp.: 94-95 °C

Analog zu der in Beispiel 83 beschriebenen Verfahrensweise lassen sich die folgenden Verbindungen der Tabelle 3 herstellen:

**Tabelle 3**

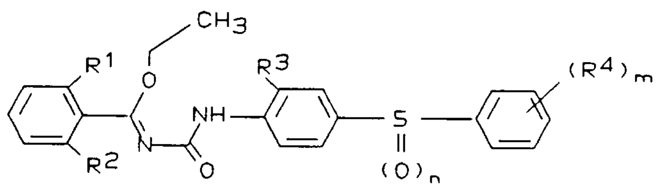 (I),

| Bsp.Nr. | $R^1$ | $R^2$ | $R^3$ | $(R^4)_m$ | n | Fp (°C) |
|---|---|---|---|---|---|---|
| 84 | F | H | H | 2-Cl, 4-$CF_3$ | 0 | 79-80 |
| 85 | F | F | H | 2-Cl, 4-$CF_3$ | 0 | 103-104 |
| 86 | Cl | H | H | 2-Cl, 4-$CF_3$ | 1 | |
| 87 | F | H | H | 2-Cl, 4-$CF_3$ | 1 | |
| 88 | F | F | H | 2-Cl, 4-$CF_3$ | 1 | |
| 89 | Cl | H | H | 2-Cl, 4-$CF_3$ | 2 | |
| 90 | F | H | H | 2-Cl, 4-$CF_3$ | 2 | |
| 91 | F | F | H | 2-Cl, 4-$CF_3$ | 2 | |
| 92 | Cl | H | F | 2-Cl, 4-$CF_3$ | 0 | |
| 93 | F | H | F | 2-Cl, 4-$CF_3$ | 0 | |
| 94 | F | F | F | 2-Cl, 4-$CF_3$ | 0 | |
| 95 | Cl | H | Cl | 2-Cl, 4-$CF_3$ | 0 | |
| 96 | F | H | Cl | 2-Cl, 4-$CF_3$ | 0 | |
| 97 | F | F | Cl | 2-Cl, 4-$CF_3$ | 0 | |
| 98 | Cl | H | H | 2-F, 4-$CF_3$ | 0 | 87-88 |
| 99 | F | H | H | 2-F, 4-$CF_3$ | 0 | 79-80 |
| 100 | F | F | H | 2-F, 4-$CF_3$ | 0 | 94-95 |
| 101 | Cl | H | F | 2-F, 4-$CF_3$ | 0 | |
| 102 | F | H | F | 2-F, 4-$CF_3$ | 0 | |
| 103 | F | F | F | 2-F, 4-$CF_3$ | 0 | |
| 104 | Cl | H | H | 2-$CF_3$, 4-Cl | 0 | |
| 105 | F | H | H | 2-$CF_3$, 4-Cl | 0 | |
| 106 | F | F | H | 2-$CF_3$, 4-Cl | 0 | |
| 107 | Cl | H | H | 3-Cl, 4-$CF_3$ | 0 | |
| 108 | F | H | H | 3-Cl, 4-$CF_3$ | 0 | |
| 109 | F | F | H | 3-Cl, 4-$CF_3$ | 0 | |
| 110 | Cl | H | H | 2-$CF_3$, 5-Cl | 0 | |
| 111 | F | H | H | 2-$CF_3$, 5-Cl | 0 | |

**Fortsetzung der Tabelle 3**

| Bsp.Nr. | $R^1$ | $R^2$ | $R^3$ | $(R^4)_m$ | n | Fp (°C) |
|---|---|---|---|---|---|---|
| 112 | F | F | H | 2-$CF_3$, 5-Cl | 0 | |
| 113 | Cl | H | H | 2,6-$Cl_2$, 4-$CF_3$ | 0 | |
| 114 | F | H | H | 2,6-$Cl_2$, 4-$CF_3$ | 0 | |
| 115 | F | F | H | 2,6-$Cl_2$, 4-$CF_3$ | 0 | |
| 116 | Cl | H | F | 2,6-$Cl_2$, 4-$CF_3$ | 0 | |
| 117 | F | H | F | 2,6-$Cl_2$, 4-$CF_3$ | 0 | |
| 118 | F | F | F | 2,6-$Cl_2$, 4-$CF_3$ | 0 | |
| 119 | Cl | H | H | 2-$NO_2$, 4-$CF_3$ | 0 | |
| 120 | F | H | H | 2-$NO_2$, 4-$CF_3$ | 0 | |
| 121 | F | F | H | 2-$NO_2$, 4-$CF_3$ | 0 | |
| 122 | Cl | H | H | 3-$CF_3$, 4-$NO_2$ | 0 | |
| 123 | F | H | H | 3-$CF_3$, 4-$NO_2$ | 0 | |
| 124 | F | F | H | 3-$CF_3$, 4-$NO_2$ | 0 | |
| 125 | Cl | H | H | 2-Cl, 4-$NO_2$ | 0 | 132-133 |
| 126 | F | H | H | 2-Cl, 4-$NO_2$ | 0 | 127-128 |
| 127 | F | F | H | 2-Cl, 4-$NO_2$ | 0 | 119-120 |
| 128 | Cl | H | H | 2,6-$(NO_2)_2$, 4-$CF_3$ | 0 | |
| 129 | F | H | H | 2,6-$(NO_2)_2$, 4-$CF_3$ | 0 | |
| 130 | F | F | H | 2,6-$(NO_2)_2$, 4-$CF_3$ | 0 | |
| 131 | Cl | H | H | 2,4-$(NO_2)_2$, 6-$CF_3$ | 0 | |
| 132 | F | H | H | 2,4-$(NO_2)_2$, 6-$CF_3$ | 0 | |
| 133 | F | F | H | 2,4-$(NO_2)_2$, 6-$CF_3$ | 0 | |
| 134 | Cl | H | H | 2,6-$(NO_2)_2$, 3-Cl, 4-$CF_3$ | 0 | |
| 135 | F | F | H | 2,6-$(NO_2)_2$, 3-Cl, 4-$CF_3$ | 0 | |

**Tabelle 4**

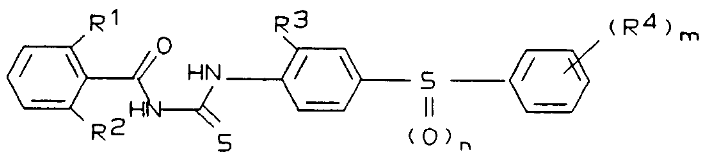

(I),

| Bsp.Nr. | $R^1$ | $R^2$ | $R^3$ | $(R^4)_m$ | n | Fp (°C) |
|---|---|---|---|---|---|---|
| 136 | Cl | H | H | 2-Cl, 4-$CF_3$ | 0 | 125-126 |
| 137 | F | H | H | 2-Cl, 4-$CF_3$ | 0 | 103-105 |
| 138 | Cl | H | F | 2-Cl, 4-$CF_3$ | 0 | 125 |
| 139 | F | F | F | 2-Cl, 4-$CF_3$ | 0 | 148 |

**C. Biologische Beispiele**

Beispiel 1 (Spodoptera-Test)

Larven des afrikanischen Baumwollwurms (Spodoptera littoralis L III) und Petrischalen, in die eine Diät auf Agarbasis eingefüllt wird, wurden in einer Spritzapparatur mit einer Wirkstoffzubereitung der Konzentration 100 ppm besprüht. Die Laven wurden nach Antrocknen des Spritzbelages auf die behandelte Agardiät gesetzt.
Nach der gewünschten Zeit (7 Tage, Häutung nach L IV) wurde die Abtötung der Raupen in % bestimmt. Dabei bedeutet 100 %, daß alle Raupen abgetötet wurden.

Bei den Verbindungen der Beispiele 1, 2, 3, 10, 12, 15, 16, 17, 18, 24, 31, 33, 37, 38, 39, 40, 42, 43, 45, 48, 50, 55, 83, 84, 85, 52b 52c, 52d, 52g bis 52q und 136-139 war bei der o.a Konzentration eine 100 %ige Mortalität festzustellen.

Beispiel 2 (Musca-Test)

24 Stunden alte Hausfliegenlarven (Musca domestica) wurden in eine Fliegendiät, in die vorher eine Wirkstoffzubereitung bis zu einer Wirkstoffkonzentration von 250 ppm eingearbeitet worden war, eingebracht.
Nach der gewünschten Zeit (L1 bis Fliegenschlupf) wurde die Abtötung der Laven bzw. der Schlupf der Fliegen in % bestimmt. Dabei bedeutet 100 %, daß alle Larven abgetötet wurden bzw. keine Fliege aus den Puppen schlüpfte.

Bei den Verbindungen 1, 2, 3, 10, 12, 15, 16, 17, 18, 24, 25, 31, 33, 37, 38, 39, 40, 41, 42, 43, 44, 45, 47, 48, 50, 55, 83, 84, 85, 52b, 52c 52d, 52e, 52f, 52h, 52i, 52k-52q und 136-139 war eine 100 %ige Wirkung bei der o.a. Konzentration zu beobachten.

Beispiel 3 (Lucilia-Test)

Die Aktivsubstanzen werden in einer Mischung bestehend aus Dimethylformamid (85 g), ®Emulsogen (7 g) und ®Arkopal N 60 (3 g) so gelöst, daß Verdünnungsreihen mit Wirkstoffkonzentrationen von 10000/1000/100 ppm erhalten werden. Jeweils 1 ml dieser Lösungen wird mit 9 g fein vermahlenem Fleisch innig vermischt, so daß letzlich Fleisch mit Wirkstoffkonzentrationen von 1000/100/10 ppm vorhanden ist. Zur Kontrolle werden 9 g Fleisch mit 1 ml des Lösungsmittels versetzt.

Den so präparierten Larvennährmedien werden jeweils 20 frisch geschlüpfte Larven, von Lucilia cuprina zugegeben. Nach 72 h, wenn sich im Kontrollmedium die Larven I zu den verpuppungsreifen Larven III entwickelt haben, wird die Mortalitätsrate ermittelt. In diesem Test erbrachten die Verbindungen gemäß

Beispiel 2, 10, 15, 16, 18, 33, 136 und 138 bei einer Wirkstoffkonzentration von 10 ppm 100 % Abtötung der Larven.

Beispiel 4 (Periplaneta-Test)

Schaben (Periplaneta germanica Larven L III) wurden in Petrischalen gesetzt, bei denen an die Innenseite des Bodens und des Deckels je 1 ml einer wäßrigen Wirkstoffzubereitung der unten genannten Verbindungen aufgetragen wurde. Dabei betrug die Wirkstoffkonzentration bezogen auf das Trägermedium Wasser 500 ppm. Vor Eingabe der Schaben war das Wasser abgetrocknet, so daß die Versuchstiere dem gleichmäßig auf der Innenseite befindlichen Wirkstoffbelag ausgesetzt waren.
Nach 5 Tagen wurden die toten Tiere bestimmt. Es zeigte sich, daß bei der o.a. Wirkstoffkonzentration bei den Verbindungen 1, 2, 16, 18, 31, 33, 37, 38, 40, 41, 42, 43, 44, 45, 55, 83, 84, 85, 52b-52f, 52h, 52i 52k-52q und 136-139 eine 100 % abtötende Wirkung verursacht wurde.

Beispiel 5 (Trialeurodes-Test)

Mit weißer Fliege (Trialeurodes vaporariorum) stark besetzte Bohnenpflanzen wurden mit wäßrien Suspensionen von Spritzpulverkonzentraten (200 ppm Wirkstoffgehalt) bis zum beginnenden Abtropfen gespritzt. Nach Aufstellung der Pflanzen im Gewächshaus erfolgte die mikroskopische Kontrolle mit dem Ergebnis jeweils 100 %iger Mortalität bei den Präparaten mit den Wirkstoffen der Beispiele 1, 2, 52h, 52j, 52l, 136 und 139.

Beispiel 6 (Tetranychus-Test)

Mit Bohnenspinnmilben (Tetranychus urticae) stark befallene Bohnenpflanzen (Phaseolus v.) wurden mit der wäßrigen Verdünnung eines Emulsionskonzentrates, des 100 ppm des jeweiligen Wirkstoffs enthielt, gespritzt.
100 % Abtötung wurde mit den Verbindungen gemäß Beispiel 1, 2, 10, 12, 15, 16, 18, 24, 25, 33, 37, 38, 39, 43, 55, 83, 84, 85, 52g bis 52n, 52p, 136-139 erzielt.

Beispiel 7 (Panonychus-Test)

Mit Obstbaumspinnmilben (Panonychus ulmi) stark befallene Apfelbäumchen wurden mit der wäßrigen Verdünnung eines Emulsionskonzentrates, das 100 ppm des jeweiligen Wirkstoffes enthielt, gespritzt.
100 %ige Abtötung wurde mit den Verbindungen gemäß Beispiel 1, 2, 3, 10, 12, 16, 18, 25, 37, 38, 39, 40, 45, 55, 83, 84, 85, 52g-52l, 52p, 136-139 erzielt.

**Patentansprüche**
**Patentansprüche für folgende Vertragsstaaten : AT, BE, CH, DE, FR, GB, GR, IT, LI, NL**

**1.** Verbindungen der Formel I

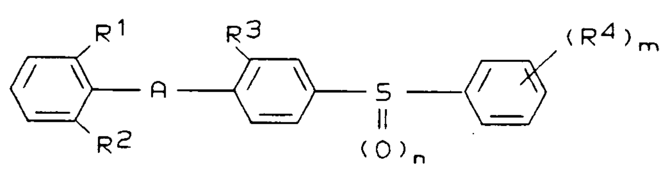

(I),

worin A einen Rest der Formeln $A^1$, $A^2$ oder $A^3$

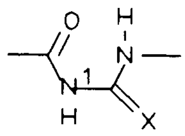 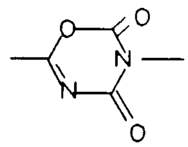 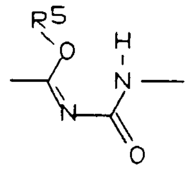

(A1) (A2) (A3) ,

| | |
|---|---|
| $R^1$ bis $R^3$ | jeweils unabhängig voneinander Wasserstoff oder Halogen, |
| $R^4$ | jeweils unabhängig von einander Halogen, $(C_1-C_3)$Halogenalkyl, oder Nitro, |
| $R^5$ | $(C_1-C_5)$Alkyl, das halogeniert sein kann, |
| X | Sauerstoff oder Schwefel, |
| n | eine Zahl von 0 bis 2 und |
| m | eine Zahl von 1 bis 4 |

bedeuten, mit der Maßgabe, daß $(R^4)_m$ nicht für sich allein Halogen oder für sich allein Nitro bedeutet, sowie im Fall $A = A^1$ deren in der Landwirtschaft einsetzbaren Salze.

**2.** Verbindungen der Formel I von Anspruch 1, worin $R^1$ = Cl, F, $R^2$ = H, F; $R^3$ = H oder F, $R^4$ = jeweils unabhängig voneinander Cl, F, $CF_3$ oder Nitro; $R^5$ = Ethyl; X = Sauerstoff; n = 0 und m = 1 bis 3 bedeuten, sowie im Fall A = $A^1$ deren in der Landwirtschaft einsetzbaren Salze.

**3.** Verbindungen der Formel I von Anspruch 1, worin $R^1$ = Cl, F und $R^2$ = H, F; $R^3$ = H oder F; $(R^4)_m$ = 2-Cl, 4-$CF_3$; $R^5$ = Ethyl; X = Sauerstoff; n = 0 m = 1 oder 2 bedeuten, sowie im Fall A = $A^1$ deren in der Landwirtschaft einsetzbaren Salze.

**4.** Die Verbindung N-(2-Fluorbenzoyl)-N'-(4-(2-chlor-4-trifluormethyl-1-phenylmercapto)phenyl)harnstoff.

**5.** Die Verbindung N-(2-Chlorbenzoyl)-N'-(4-(2-chlor-4-trifluormethyl-1-phenylmercapto)phenyl)harnstoff.

**6.** Die Verbindung N-(2,6-Difluorbenzoyl)-N'-(4-(2-chlor-4-trifluormethyl-1-phenylmercapto)phenyl)harnstoff.

**7.** Die Verbindung N-(N-(2-Chlor-4-trifluormethyl-1-phenylmercapto)phenyl)carbamoyl)-2-fluorbenzcarboximidsäureethylester.

**8.** Die Verbindung N-(N-(2-Chlor-4-trifluormethyl-1-phenylmercapto)phenyl)carbamoyl)-2-chlorbenzcarboximidsäureethylester.

**9.** Die Verbindung N-(N-(2-Chlor-4-trifluormethyl-1-phenylmercapto)phenyl)carbamoyl)-2,6-difluorbenzcarboximidsäureethylester.

**10.** Die Verbindung N-(2-Chlorbenzoyl)-N'-(4-(2-chlor-4-trifluormethyl-1-phenylmercapto)-2-fluorphenyl)-harnstoff.

**11.** Die Verbindung N-(2-Fluorbenzoyl)-N'-(4-(2-chlor-4-trifluormethyl-1-phenylmercapto)-2-fluorphenyl)-harnstoff.

**12.** Die Verbindung N-(2,6-Difluorbenzoyl)-N'-(4-(2-chlor-4-trifluormethyl)-1-phenylmercapto)-2-fluorphenyl)-harnstoff.

**13.** Verfahren zur Herstellung von Verbindungen der Formel I gemäß einem oder mehreren der Ansprüche 1-9, dadurch gekennzeichnet, daß man
a) für A = $A^1$

$a_1$) eine Verbindung der Formel II

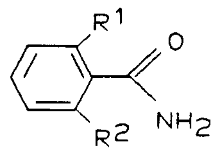

(II),

worin $R^1$ und $R^2$ die Bedeutungen wie in Formel I besitzen, mit einer Verbindung der Formel III

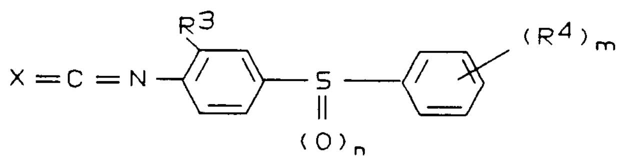

(III),

worin X, $R^3$, $R^4$, m und n die Bedeutungen wie in Formel I besitzen, umsetzt oder
$a_2$) eine Verbindung der Formel IV

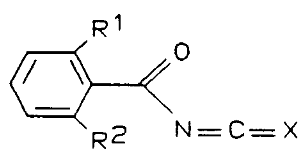

(IV),

worin $R^1$, $R^2$ und X die Bedeutungen wie in Formel I besitzen, mit einer Verbindung der Formel V

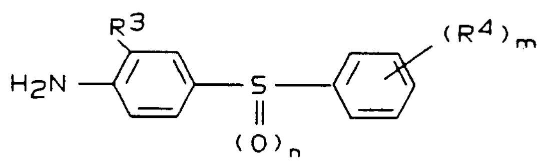

(V),

worin $R^3$, $R^4$, m und n die Bedeutungen wie in Formel I besitzen, umsetzt
oder
b) für $A = A^2$
eine Verbindung der Formel III mit einer Verbindung der Formel IV, wobei X jeweils 0 bedeutet, umsetzt
oder
c) für $A = A^3$
$c_1$) eine Verbindung der Formel VI

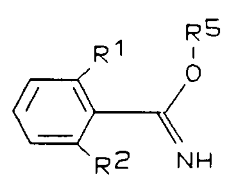

(VI),

worin $R^1$, $R^2$ und $R^5$ die Bedeutungen wie in Formel I besitzen, mit einer Verbindung der Formel III umsetzt
oder

$c_2$) eine Verbindung der Formel VII

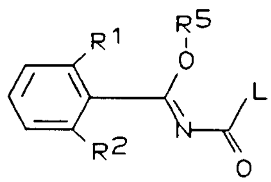

(VII),

worin $R^1$, $R^2$ und $R^4$ die Bedeutungen wie in Formel I besitzen und L für eine nucleofuge Abgangsgruppe steht, mit einer Verbindung der Formel V umsetzt.

**14.** Akarzide oder insektizide Mittel, dadurch gekennzeichnet, daß sie eine wirksame Menge einer Verbindung der Formel I enthalten.

**15.** Verwendung der Verbindungen der Formel I zur Bekämpfung von Akariden oder Schadinsekten.

**16.** Verfahren zur Bekämpfung von Akariden oder Schadinsekten, dadurch gekennzeichnet, daß man auf diese oder die von ihnen befallenen Pflanzen, Flächen oder Substrate eine wirksame Menge einer Verbindung der Formel I appliziert.

**Patentansprüche für folgenden Vertragsstaat: ES**

**1.** Verfahren zur Herstellung von Verbindungen der Formel I

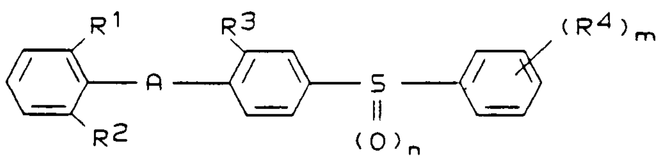

(I),

worin A einen Rest der Formeln $A^1$, $A^2$ oder $A^3$

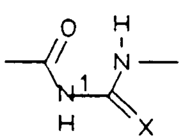

(A1)

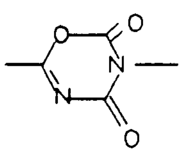

(A2)

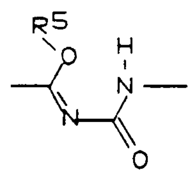

(A3)

,

| | |
|---|---|
| $R^1$ bis $R^3$ | jeweils unabhängig voneinander Wasserstoff oder Halogen, |
| $R^4$ | jeweils unabhängig von einander Halogen, $(C_1-C_3)$Halogenalkyl oder Nitro, |
| $R^5$ | $(C_1-C_5)$ Alkyl, das halogeniert sein kann, |
| X | Sauerstoff oder Schwefel, |
| n | eine Zahl von 0 bis 2 und |
| m | eine Zahl von 1 bis 4 |

bedeuten, mit der Maßgabe, daß $(R^4)_m$ nicht für sich allein Halogen oder für sich allein Nitro bedeutet, sowie im Fall $A = A^1$ deren in der Landwirtschaft einsetzbaren Salze, dadurch gekennzeichnet, daß man

a) für $A = A^1$

$a_1$) eine Verbindung der Formel II

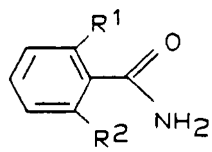

(II),

worin $R^1$ und $R^2$ die Bedeutungen wie in Formel I besitzen, mit einer Verbindung der Formel III

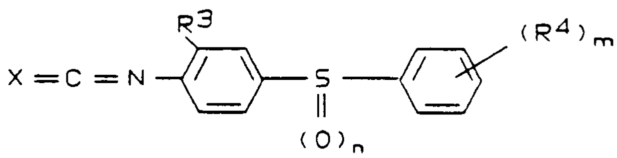

(III),

worin X, $R^3$, $R^4$, m und n die Bedeutungen wie in Formel I besitzen, umsetzt oder
$a_2$) eine Verbindung der Formel IV

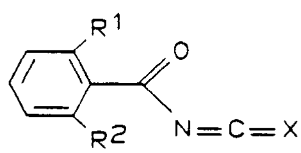

(IV),

worin $R^1$, $R^2$ und X die Bedeutungen wie in Formel I besitzen, mit einer Verbindung der Formel V

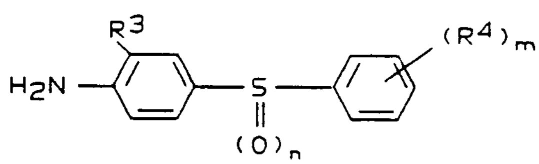

(V),

worin $R^3$, $R^4$, m und n die Bedeutungen wie in Formel I besitzen, umsetzt
oder
b) für A = $A^2$
eine Verbindung der Formel III mit einer Verbindung der Formel IV, wobei X jeweils 0 bedeutet, umsetzt
oder
c) für A = $A^3$
$c_1$) eine Verbindung der Formel VI

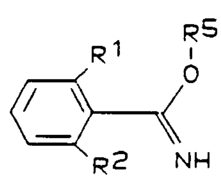

(VI),

worin $R^1$, $R^2$ und $R^5$ die Bedeutungen wie in Formel I besitzen, mit einer Verbindung der Formel III umsetzt
oder

$c_2$) eine Verbindung der Formel VII

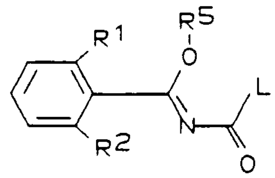

(VII),

worin $R^1$, $R^2$ und $R^4$ die Bedeutungen wie in Formel I besitzen und L für eine nucleofuge Abgangsgruppe steht, mit einer Verbindung der Formel V umsetzt.

**2.** Verfahren gemäß Anspruch 1, dadurch gekennzeichnet, daß in Formel I $R^1$ = Cl, F, $R^2$ = H, F; $R^3$ = H oder F, $R^4$ = jeweils unabhängig voneinander Cl, F, $CF_3$ oder Nitro; $R^5$ = Ethyl; X = Sauerstoff; n = 0 und m = 1 bis 3 bedeuten, sowie im Fall A = $A^1$ deren in der Landwirtschaft einsetzbaren Salze.

**3.** Verfahren gemäß Anspruch 1, dadurch gekennzeichnet, daß in Formel I $R^1$ = Cl, F und $R^2$ = H, F; $R^3$ = H oder F; $(R^4)_m$ = 2-Cl, 4-$CF_3$; $R^5$ = Ethyl; X = Sauerstoff; n = 0 und m = 1 oder 2 bedeuten, sowie im Fall A = $A^1$ deren in der Landwirtschaft einsetzbaren Salze.

**4.** Verfahren gemäß Anspruch 1, dadurch gekennzeichnet, daß Formel I für N-(2-Fluorbenzoyl)-N'-(4-(2-chlor-4-trifluormethyl-1-phenylmercapto)phenyl)harnstoff steht.

**5.** Verfahren gemäß Anspruch 1, dadurch gekennzeichnet, daß Formel I für N-(2-Chlorbenzoyl)-N'-(4-(2-chlor-4-trifluormethyl-1-phenylcercapto)phenyl)harnstoff steht.

**6.** Verfahren gemäß Anspruch 1, dadurch gekennzeichnet, daß Formel I für N-(2,6-Difluorbenzoyl)-N'-(4-(2-chlor-4-trifluormethyl-1-phenylmercapto)phenyl)harnstoff steht.

**7.** Verfahren gemäß Anspruch 1, dadurch gekennzeichnet, daß Formel I für N-(N-(2-Chlor-4-trifluormethyl-1-phenylmercapto)phenyl)carbamoyl)-2-fluorbenzcarboximidsäureethylester steht.

**8.** Verfahren gemäß Anspruch 1, dadurch gekennzeichnet, daß Formel I für N-(N-(2-Chlor-4-trifluormethyl-1-phenylmercapto)phenyl)carbamoyl)-2-chlorbenzcarboximidsäureethylester steht.

**9.** Verfahren gemäß Anspruch 1, dadurch gekennzeichnet, daß Formel I für N-(N-(2-Chlor-4-trifluormethyl-1-phenylmercapto)phenyl)carbamoyl)-2,6-difluorbenzcarboximidsäureethylester steht.

**10.** Verfahren gemäß Anspruch 1, dadurch gekennzeichnet, daß Formel I für N-(2-Chlorbenzoyl)-N'-(4-(2-chlor-4-trifluormethyl-1-phenylmercapto)-2-fluorphenyl)harnstoff steht.

**11.** Verfahren gemäß Anspruch 1, dadurch gekennzeichnet, daß Formel I für N-(2-Fluorbenzoyl)-N'-(4-(2-chlor-4-trifluormethyl-1-(phenylmercapto)-2-fluorphenyl)harnstoff steht.

**12.** Verfahren gemäß Anspruch 1, dadurch gekennzeichnet, daß Formel I für N-(2,6-Difluorbenzoyl)-N'-(4-(2-chlor-4-trifluormethyl)-1-phenylmercapto)-2-fluorphenyl)harnstoff steht.

**13.** Akarzide oder insektizide Mittel, dadurch gekennzeichnet, daß sie eine wirksame Menge einer Verbindung der Formel I gemäß Ansprüchen 1-12 enthalten.

**14.** Verwendung der Verbindungen der Formel I gemäß Ansprüchen 1-12 zur Bekämpfung von Akariden oder Schadinsekten.

**15.** Verfahren zu Bekämpfung von Akariden oder Schadinsekten, dadurch gekennzeichnet, daß man auf diese oder die von ihnen befallenen Pflanzen, Flächen oder Substrate eine wirksame Menge einer Verbindung der Formel I gemäß Ansprüchen 1-12 appliziert.

## Claims

**Claims for the following Contracting States : AT, BE, CH, DE, FR, GB, GR, IT, LI, NL**

1. A compound of the formula I

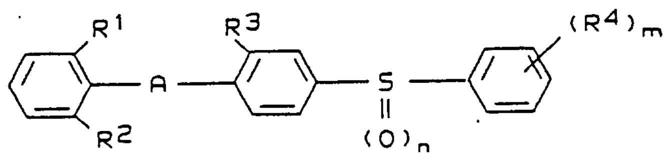

(I)

in which A denotes a radical of the formula $A^1$, $A^2$ or $A^3$

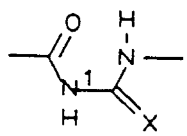

($A^1$)

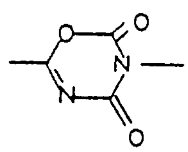

($A^2$)

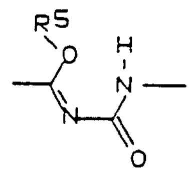

($A^3$)

| | |
|---|---|
| $R^1$ to $R^3$ | in each case independently of one another denote hydrogen or halogen, |
| the radicals $R^4$ | in each case independently of one another denote halogen, $(C_1\text{-}C_3)$halogenoalkyl or nitro, |
| $R^5$ | denotes $(C_1\text{-}C_5)$alkyl, which can be halogenated, |
| X | denotes oxygen or sulfur, |
| n | denotes a number from 0 to 2 and |
| m | denotes a number from 1 to 4 |

with the proviso that $(R^4)_m$ does not by itself denote halogen or by itself denote nitro, or where $A = A^1$, a salt thereof which can be used in agriculture.

2. A compound of the formula I as claimed in claim 1, in which $R^1$ denotes Cl or F and $R^2$ denotes H or F; $R^3$ denotes H or F and the radicals $R^4$ in each case independently of one another denote Cl, F, $CF_3$ or nitro; $R^5$ denotes ethyl; X denotes oxygen; n denotes 0 and m denotes 1 to 3, and where $A = A^1$, a salt thereof which can be used in agriculture.

3. A compound of the formula I as claimed in claim 1, in which $R^1$ denotes Cl or F and $R^2$ denotes H or F; $R^3$ denotes H or F and $(R^4)_m$ denotes 2-Cl or 4-$CF_3$; $R^5$ denotes ethyl; X denotes oxygen, n denotes 0 and m denotes 1 or 2, and where $A = A^1$, a salt thereof which can be used in agriculture.

4. The compound N-(2-fluorobenzoyl)-N'-(4-(2-chloro-4-trifluoromethyl-1-phenylmercapto)phenyl)urea.

5. The compound N-(2-chlorobenzoyl)-N'-(4-(2-chloro-4-trifluoromethyl-1-phenylmercapto)phenyl)urea.

6. The compound N-(2,6-difluorobenzoyl)-N'-(4-(2-chloro-4-trifluoromethyl-1-phenylmercapto)phenyl)urea.

7. The compound ethyl N-(N-(2-chloro-4-trifluoromethyl-1-phenylmercapto)phenyl)carbamoyl)-2-fluorobenzocarboximidate.

8. The compound ethyl N-(N-(2-chloro-4-trifluoromethyl-1-phenylmercapto)phenyl)carbamoyl)-2-chlorobenzocarboximidate.

9. The compound ethyl N-(N-(2-chloro-4-trifluoromethyl-1-phenylmercapto)phenyl)carbamoyl)-2,6-difluorobenzocarboximidate.

**10.** The compound N-(2-chlorobenzoyl)-N'-(4-(2-chloro-4-trifluoromethyl-1-phenylmercapto)-2-fluorophenyl)-urea.

**11.** The compound N-(2-fluorobenzoyl)-N'-(4-(2-chloro-4-trifluoromethyl-1-phenylmercapto)-2-fluorophenyl)-urea.

**12.** The compound N-(2,6-difluorobenzoyl)-N'-(4-(2-chloro-4-trifluoromethyl)-1-phenylmercapto)-2-fluorophenyl)urea.

**13.** A process for the preparation of a compound of the formula I as claimed in one or more of claims 1 - 9, which comprises

a) where A = $A^1$

$a_1$) reacting a compound of the formula II

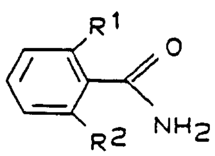

(II)

in which $R^1$ and $R^2$ have the meanings as in formula I, with a compound of the formula III

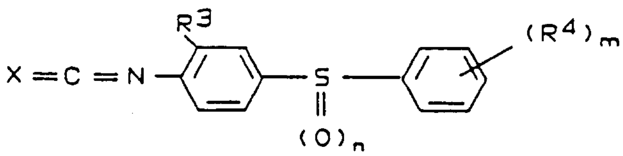

(III)

in which X, $R^3$, $R^4$, m and n have the meanings as in formula I, or

$a_2$) reacting a compound of the formula IV

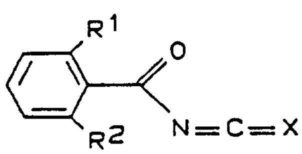

(IV)

in which $R^1$, $R^2$ and X have the meanings as in formula I, with a compound of the formula V

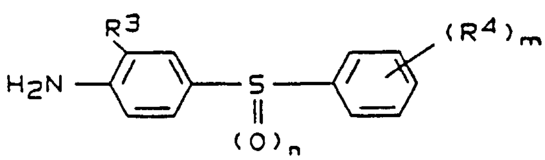

(V)

in which $R^3$, $R^4$, m and n have the meanings as in formula I,

or

b) where A = $A^2$

reacting a compound of the formula III with a compound of the formula IV, in which X in each case denotes 0,

or

c) where A = $A^3$

$c_1$) reacting a compound of the formula VI

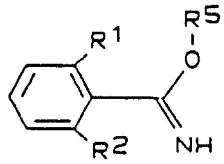

(VI)

in which $R^1$, $R^2$ and $R^5$ have the meanings as in formula I, with a compound of the formula III, or

$c_2$) reacting a compound of the formula VII

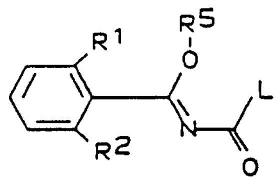

(VII)

in which $R^1$, $R^2$ and $R^5$ have the meanings as in formula I and L stands for a nucleofugic leaving group, with a compound of the formula V.

**14.** An acaricidal or insecticidal agent which contains an effective amount of a compound of the formula I.

**15.** The use of a compound of the formula I for combating acarides or harmful insects.

**16.** A method of combating acarides or harmful insects, which comprises applying an effective amount of a compound of the formula I to these or to the plants, areas or substrates affected by them.

**Claims for the following Contracting State : ES**

**1.** A process for the preparation of a compound of the formula I

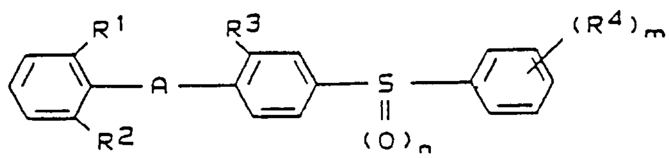

(I)

in which A denotes a radical of the formula $A^1$, $A^2$ or $A^3$

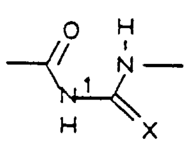

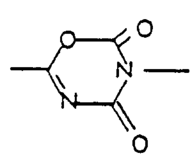

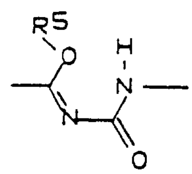

($A^1$) ($A^2$) ($A^3$)

| | |
|---|---|
| $R^1$ to $R^3$ | in each case independently of one another denote hydrogen or halogen, |
| the radicals $R^4$ | in each case independently of one another denote halogen, $(C_1\text{-}C_3)$halogenoalkyl or nitro, |
| $R^5$ | denotes $(C_1\text{-}C_5)$alkyl, which can be halogenated, |

X denotes oxygen or sulfur,
n denotes a number from 0 to 2 and
m denotes a number from 1 to 4

with the proviso that $(R^4)_m$ does not by itself denote halogen or by itself denote nitro, or where $A = A^1$, a salt thereof which can be used in agriculture, which comprises

a) where $A = A^1$

$a_1$) reacting a compound of the formula II

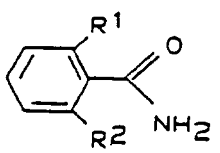

(II)

in which $R^1$ and $R^2$ have the meanings as in formula I, with a compound of the formula III

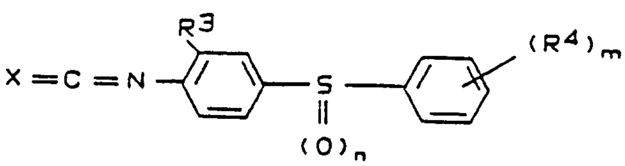

(III)

in which X, $R^3$, $R^4$, m and n have the meanings as in formula I, or

$a_2$) reacting a compound of the formula IV

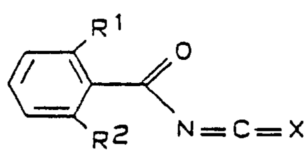

(IV)

in which $R^1$, $R^2$ and X have the meanings as in formula I, with a compound of the formula V

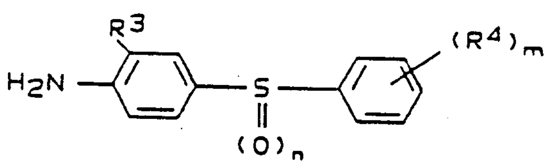

(V)

in which $R^3$, $R^4$, m and n have the meanings as in formula I,

or

b) where $A = A^2$

reacting a compound of the formula III with a compound of the formula IV, in which X in each case denotes 0,

or

c) where $A = A^3$

$c_1$) reacting a compound of the formula VI

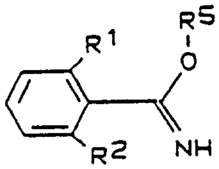

(VI)

in which $R^1$, $R^2$ and $R^5$ have the meanings as in formula I, with a compound of the formula III, or

$c_2$) reacting a compound of the formula VII

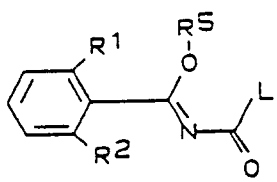

(VII)

in which $R^1$, $R^2$ and $R^5$ have the meanings as in formula I and L stands for a nucleofugic leaving group, with a compound of the formula V.

**2.** The process as claimed in claim 1, wherein in formula I $R^1$ denotes Cl or F and $R^2$ denotes H or F; $R^3$ denotes H or F and the radicals $R^4$ in each case independently of one another denote Cl, F, $CF_3$ or nitro; $R^5$ denotes ethyl; X denotes oxygen; n denotes 0 and m denotes 1 to 3, and where A = $A^1$, a salt thereof which can be used in agriculture.

**3.** The process as claimed in claim 1, wherein in formula I $R^1$ denotes Cl or F and $R^2$ denotes H or F; $R^3$ denotes H or F and $(R^4)_m$ denotes 2-Cl or 4-$CF_3$; $R^5$ denotes ethyl; X denotes oxygen, n denotes 0 and m denotes 1 or 2, and where A = $A^1$, a salt thereof which can be used in agriculture.

**4.** The process as claimed in claim 1, wherein formula I represents N-(2-fluorobenzoyl)-N'-(4-(2-chloro-4-trifluoromethyl-1-phenylmercapto)phenyl)urea.

**5.** The process as claimed in claim 1, wherein formula I represents N-(2-chlorobenzoyl)-N'-(4-(2-chloro-4-trifluoromethyl-1-phenylmercapto)phenyl)urea.

**6.** The process as claimed in claim 1, wherein formula I represents N-(2,6-difluorobenzoyl)-N'-(4-(2-chloro-4-trifluoromethyl-1-phenylmercapto)phenyl)urea.

**7.** The process as claimed in claim 1, wherein formula I represents ethyl N-(N-(2-chloro-4-trifluoromethyl-1-phenylmercapto)phenyl)carbamoyl)-2-fluorobenzocarboximidate.

**8.** The process as claimed in claim 1, wherein formula I represents ethyl N-(N-(2-chloro-4-trifluoromethyl-1-phenylmercapto)phenyl)carbamoyl)-2-chlorobenzocarboximidate.

**9.** The process as claimed in claim 1, wherein formula I represents ethyl N-(N-(2-chloro-4-trifluoromethyl-1-phenylmercapto)phenyl)carbamoyl)-2,6-difluorobenzocarboximidate.

**10.** The process as claimed in claim 1, wherein formula I represents N-(2-chlorobenzoyl)-N'-(4-(2-chloro-4-trifluoromethyl-1-phenylmercapto)-2-fluorophenyl)urea.

**11.** The process as claimed in claim 1, wherein formula I represents N-(2-fluorobenzoyl)-N'-(4-(2-chloro-4-trifluoromethyl-1-phenylmercapto)-2-fluorophenyl)urea.

**12.** The process as claimed in claim 1, wherein formula I represents N-(2,6-difluorobenzoyl)-N'-(4-(2-chloro-4-trifluoromethyl)-1-phenylmercapto)-2-fluorophenyl)urea.

**13.** An acaricidal or insecticidal agent which contains an effective amount of a compound of the formula I as claimed in claims 1 - 12.

**14.** The use of a compound of the formula I as claimed in claims 1 - 12 for combating acarides or harmful insects.

**15.** A method of combating acarides or harmful insects, which comprises applying an effective amount of a compound of the formula I as claimed in claims 1 - 12 to these or to the plants, areas or substrates affected by them.

**Revendications**

**Revendications pour les Etats contractants suivants : AT, BE, CH, DE, FR, GB, GR, IT, LI, NL**

**1.** Composés de formule I

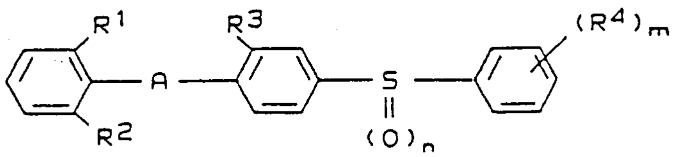

(I)

dans laquelle A représente un radical de formule $A^1$, $A^2$ ou $A^3$

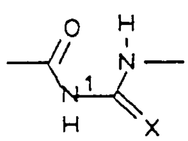

(A1)

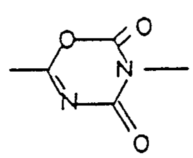

(A2)

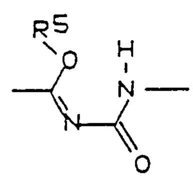

(A3)

| | |
|---|---|
| $R^1$ à $R^3$ | représentent chacun, indépendamment les uns des autres, l'hydrogène ou un halogène, |
| les symboles $R^4$ | chacun, indépendamment les uns des autres, un halogène, un halogénoalkyle en $C_1$-$C_3$ ou le groupe nitro, |
| $R^5$ | désigne un alkyle en $C_1$-$C_5$ pouvant être halogéné, |
| X | l'oxygène ou le soufre, |
| n | un nombre de 0 à 2 et |
| m | un nombre de 1 à 4, |

avec la condition que $(R^4)_m$ ne soit pas exclusivement un halogène ou le groupe nitro , ainsi que, si A est un radical $A^1$, les sels de ces composés utilisables en agriculture.

**2.** Composés de formule I de la revendication 1 dans lesquels $R^1$ est le chlore ou le fluor, $R^2$ l'hydrogène ou le fluor, $R^3$ l'hydrogène ou le fluor, $R^4$, indépendamment l'un de l'autre, le chlore ou le fluor ou un groupe $-CF_3$ ou nitro, $R^5$ est le groupe éthyle, X l'oxygène, n le nombre 0 et m un nombre de 1 à 3, ainsi que, dans le cas où A est un radical $A^1$, leurs sels utilisables en agriculture.

**3.** Composés de formule I de la revendication 1 dans lesquels $R^1$ est le chlore ou le fluor, $R^2$ et $R^3$ sont chacun l'hydrogène ou le fluor, $(R^4)_m$ représente un atome de chlore à la position 2 et/ou un groupe $-CF_3$ à la position 4, $R^5$ est le groupe éthyle, X l'oxygène, n le nombre 0 et m le nombre de 1 ou 2, ainsi que, dans le cas où A est un radical $A^1$, leurs sels utilisables en agriculture.

**4.** N-(2-chlorobenzoyl)-N'-(4-(2-chloro-4-trifluorométhyl-1-phénylmercapto)phényl)urée.

**5.** N-(2-fluorobenzoyl)-N'-(4-(2-chloro-4-trifluorométhyl-1-phénylmercapto)phényl)urée.

**6.** N-(2,6-difluorobenzoyl)-N'-(4-(2-chloro-4-trifluorométhyl-1-phénylmercapto)phényl)urée.

**7.** N-(2-chlorobenzoyl)-N'-(4-(2-chloro-4-trifluorométhyl-1-phénylmercapto)-2-fluorophényl)urée.

**8.** N-(2-fluorobenzoyl)-N'-(4-(2-chloro-4-trifluorométhyl-1-phénylmercapto)-2-fluorophényl)urée.

**9.** N-(2,6-difluorobenzoyl)-N'-(4-(2-chloro-4-trifluorométhyl-1-phénylmercapto)-2-fluorophényl)urée.

**10.** N-(N-(2-chloro-4-trifluorométhyl-1-phénylmercapto)phényl)carbamoyl)-2-chlorobenzocarboximidate d'éthyle.

**11.** N-(N-(2-chloro-4-trifluorométhyl-1-phénylmercapto)phényl)carbamoyl)-2-fluorobenzocarboximidate d'éthyle.

**12.** N-(N-(2-chloro-4-trifluorométhyl-1-phénylmercapto)phényl)carbamoyl)-2,6-difluorobenzocarboximidate d'éthyle.

**13.** Procédé de préparation de composés de formule I selon une ou plusieurs des revendications 1 à 12, procédé caractérisé en ce que :

a) si A est un radical $A^1$,

$a_1$) on fait réagir un composé de formule II

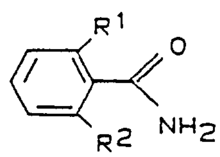

(II)

avec un composé de formule III

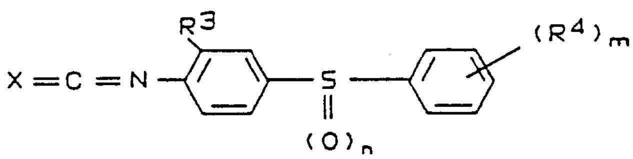

(III)

ou

$a_2$) on fait réagir un composé de formule IV

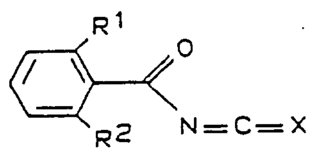

(IV)

avec un composé de formule V

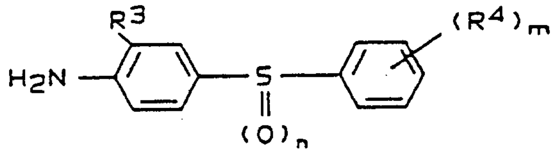

(V)

ou bien
b) si A est un radical $A^2$,
on fait réagir un composé de formule III ci-dessus avec un composé de formule IV dans lesquels X est l'oxygène,
ou encore
c) si A est un radical $A^3$,
$c_1$) on fait réagir un composé de formule VI

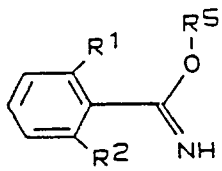

(VI)

avec un composé de formule III,
ou
$c_2$) un composé de formule VII

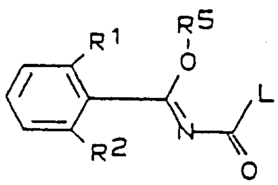

(VII)

L désignant un radical nucléofuge qui s'élimine, avec un composé de formule V.

**14.** Produits acaricides ou insecticides caractérisés en ce qu'ils contiennent une proportion efficace d'un composé de formule I selon la revendication 1.

**15.** Emploi des composés de formule I selon la revendication 1 pour combattre des acariens ou insectes nuisibles.

**16.** Procédé de lutte contre des acariens ou insectes nuisibles, procédé caractérisé en ce qu'on leur applique, ou bien sur les plantes qu'ils attaquent, surfaces ou autres substrats, une quantité efficace d'un composé de formule I selon la revendication 1.

**Revendications pour l'Etat contractant suivant : ES**

**1.** Procédé de préparation de composés de formule I

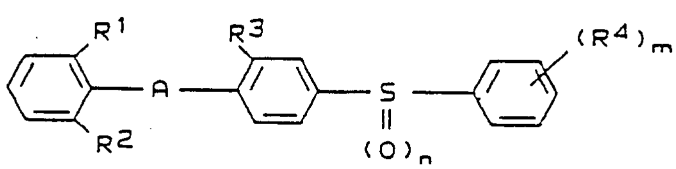

(I)

dans laquelle A représente un radical de formule $A^1$, $A^2$ ou $A^3$

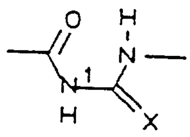

(A1)

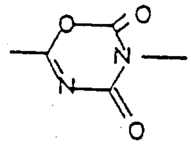

(A2)

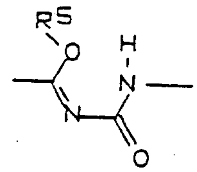

(A3)

| | |
|---|---|
| $R^1$ à $R^3$ | représentent chacun, indépendamment les uns des autres, l'hydrogène ou un halogène, |
| les symboles $R^4$ | chacun, indépendamment les uns des autres, un halogène, un halogénoalkyle en $C_1$-$C_3$ ou le groupe nitro, |
| $R^5$ | désigne un alkyle en $C_1$-$C_5$ pouvant être halogéné, |
| X | l'oxygène ou le soufre, |
| n | un nombre de 0 à 2 et |
| m | un nombre de 1 à 4, |

avec la condition que $(R^4)_m$ ne soit pas exclusivement un halogène ou le groupe nitro , ainsi que, si A est un radical $A^1$, de sels de ces composés utilisables en agriculture,

procédé caractérisé en ce que :

a) si A est un radical $A^1$,

$a_1$) on fait réagir un composé de formule II

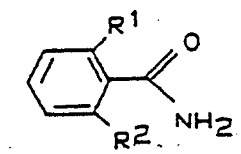

(II)

avec un composé de formule III

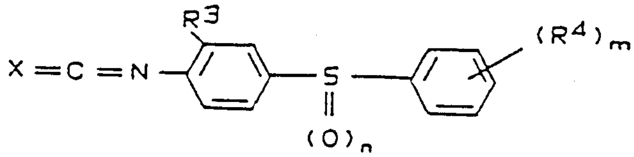

(III)

ou

$a_2$) on fait réagir un composé de formule IV

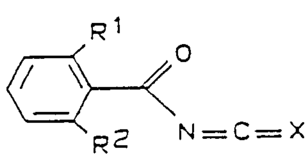

(IV)

avec un composé de formule V

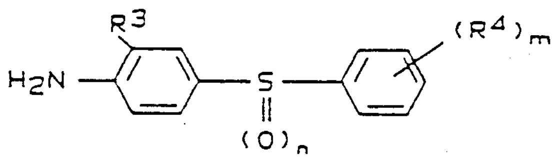

(V)

ou bien

b) si A est un radical $A^2$,

on fait réagir un composé de formule III ci-dessus avec un composé de formule IV dans lesquels X est l'oxygène,

ou encore

c) si A est un radical $A^3$,

$c_1$) on fait réagir un composé de formule VI

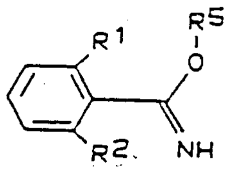

(VI)

avec un composé de formule III,

ou

$c_2$) un composé de formule VII

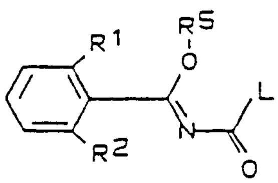

(VII)

L désignant un radical nucléofuge qui s'élimine, avec un composé de formule V.

2. Procédé selon la revendication 1, caractérisé en ce que dans la formule I $R^1$ est le chlore ou le fluor, $R^2$ l'hydrogène ou le fluor, $R^3$ l'hydrogène ou le fluor, $R^4$, indépendamment l'un de l'autre, le chlore ou le fluor ou un groupe $-CF_3$ ou nitro, $R^5$ est le groupe éthyle, X l'oxygène, n le nombre 0 et m un nombre de 1 à 3.

3. Procédé selon la revendication 1, caractérisé en ce que dans la formule I $R^1$ est le chlore ou le fluor, $R^2$ et $R^3$ sont chacun l'hydrogène ou le fluor, $(R^4)_m$ représente un atome de chlore à la position 2 et/ou un groupe $-CF_3$ à la position 4, $R^5$ est le groupe éthyle, X l'oxygène, n le nombre 0 et m le nombre de 1 ou 2.

4. Procédé selon la revendication 1, caractérisé en ce que le composé de formule I est le composé N-(2-chlorobenzoyl)-N'-(4-(2-chloro-4-trifluorométhyl-1-phénylmercapto)phényl)urée.

5. Procédé selon la revendication 1, caractérisé en ce que le composé de formule I est le composé N-(2-fluorobenzoyl)-N'-(4-(2-chloro-4-trifluorométhyl-1-phénylmercapto)phényl)urée.

6. Procédé selon la revendication 1, caractérisé en ce que le composé de formule I est le composé N-(2,6-difluorobenzoyl)-N'-(4-(2-chloro-4-trifluorométhyl-1-phénylmercapto)phényl)urée.

7. Procédé selon la revendication 1, caractérisé en ce que le composé de formule I est le composé N-(2-chlorobenzoyl)-N'-(4-(2-chloro-4-trifluorométhyl-1-phénylmercapto)-2-fluorophényl)urée.

8. Procédé selon la revendication 1, caractérisé en ce que le composé de formule I est le composé N-(2-fluorobenzoyl)-N'-(4-(2-chloro-4-trifluorométhyl-1-phénylmercapto)-2-fluorophényl)urée.

9. Procédé selon la revendication 1, caractérisé en ce que le composé de formule I est le composé N-(2,6-difluorobenzoyl)-N'-(4-(2-chloro-4-trifluorométhyl-1-phénylmercapto)-2-fluorophényl)urée.

10. Procédé selon la revendication 1, caractérisé en ce que le composé de formule I est le composé N-(N-(2-chloro-4-trifluorométhyl-1-phénylmercapto)phényl)carbamoyl)-2-chlorobenzocarboximidate d'éthyle.

11. Procédé selon la revendication 1, caractérisé en ce que le composé de formule I est le composé N-(N-(2-chloro-4-trifluorométhyl-1-phénylmercapto)phényl)carbamoyl)-2-fluorobenzocarboximidate d'éthyle.

12. Procédé selon la revendication 1, caractérisé en ce que le composé de formule I est le composé N-(N-(2-chloro-4-trifluorométhyl-1-phénylmercapto)phényl)carbamoyl)-2,6-difluorobenzocarboximidate d'éthyle.

13. Produits acaricides ou insecticides caractérisés en ce qu'ils contiennent une proportion efficace d'un composé de formule I selon les revendications 1 à 12.

14. Emploi des composés de formule I selon les revendications 1 à 12 pour combattre des acariens ou insectes nuisibles.

15. Procédé de lutte contre des acariens ou insectes nuisibles, procédé caractérisé en ce qu'on leur applique, ou bien sur les plantes qu'ils attaquent, surfaces ou autres substrats, une quantité efficace d'un composé de formule I selon les revendications 1 à 12.